# EUROPEAN PATENT APPLICATION

(11) **EP 4 357 352 A1**
(43) Date of publication of application: **24.04.2024**
(21) Application number: 22383017.5
(22) Date of filing: 21.10.2022
(51) Int. Cl.: C07H 3/04, C07H 3/06, C07H 1/08, A23L 27/30

(54) **OLIGOSACCHARIDES DERIVED FROM RARE SUGARS WITH LOW CALORIC POWER AND PREBIOTIC PROPERTIES**

(71) Applicant: Consejo Superior De Investigaciones Científicas, 28006 Madrid (ES)
(72) Inventor: Hernández Hernández, Oswaldo Jesús, Madrid (ES); García Doyagüez, Elisa, Madrid (ES); De las Rivas González del Rey, Blanca, Madrid (ES); Muñoz Moreno, M. Rosario, Madrid (ES); Muñoz Labrador,, Ana María, Madrid (ES); Calvete de la Torre, Inés, Madrid (ES); Sabater Sánchez, Carlos, Madrid (ES); Ruiz García, Lorena, Madrid (ES); Margolles Barros, Abelardo, Madrid (ES); Mancheño Gómez, José Miguel, Madrid (ES); Moreno Andújar, Francisco Javier, Madrid (ES)
(74) Representative: Pons

(57) **Abstract**

The invention relates to an oligosaccharide of formula I derived from rare sugars, wherein R, X and n have the meanings defined in the specification. The invention also relates to its process to obtain it and to its use as a prebiotic and low caloric sweetener.

[β-D-fructofuranoside(2→6)]ₙ-β-D-fructofuranoside-(2→X)-R Formula **I**

## Description

The invention relates to an oligosaccharide of formula I derived from rare sugars. The invention also relates to its process to obtain it and to its use as a prebiotic and low caloric sweetener.

### BACKGROUND ART

According to the International Society of Rare Sugars (ISRS), rare sugars are monosaccharides and their derivatives with limited availability in nature (Granström TB, Takata G, Tokuda M, Izumori K. Izumoring: a novel and complete strategy for bioproduction of rare sugars. J Biosci Bioeng. 2004;97(2):89-94). Ketohexoses such as tagatose, sorbose or psicose (both in D- and L- configurations), polyols as xylitol, or deoxygenated monosaccharides as L-ribose, are the main groups of rare sugars. Despite their low natural abundance, rare sugars stand for an enormous potential for practical applications in the food, pharmaceutical and nutrition industries because of their biological functions.

Particularly, the rare sugar D-Tagatose has physical properties similar to those of sucrose in terms of color, texture and sweetness but its glycemic index and caloric value seem to be much lower because of low bioavalability (Bober JR, Nair NU. Galactose to tagatose isomerization at moderate temperatures with high conversion and productivity. Nat Commun. 2019 Oct 7;10(1):4548). In consequence, its properties as a low-calorie sweetener point out D-tagatose as the most demanded and commercially important rare sugar.

On the other hand, tagatose has been also shown prebiotic properties (see, for example, Levin GV. Tagatose, the new GRAS sweetener and health product. J Med Food. 2002 Spring;5(1):23-36). However, the role of tagatose as a prebiotic ingredient could be seriously undermined by studies that have shown D-tagatose to be absorbed at least to some extent in the small intestine in rats (Saunders JP, Zehner LR, Levin GV. Disposition of D-[U-14C]tagatose in the rat. Regul Toxicol Pharmacol. 1999 Apr;29(2 Pt 2):S46-56) and to a greater extent in humans (Normén L, Laerke HN, Jensen BB, Langkilde AM, Andersson H. Small-bowel absorption of D-tagatose and related effects on carbohydrate digestibility: an ileostomy study. Am J Clin Nutr. 2001 Jan;73(1):105-10). Thus, the human study indicated a mean absorption rate of 80% (range 69-88%) in the small intestine and a urinary excretion of either 1% or 5%, so only the remaining 20% of D-tagatose could be fermented in the colon.

Accordingly, it would be desirable to obtain novel non-digestible rare sugars-based oligosaccharides exerting physical properties like those of sucrose but also a remarkable refinement since this oligosaccharide would likely resist gastrointestinal digestion and pass through the small intestine, becoming available for consumption by the colonic microbiota at a greater extent than D-tagatose.

### SUMMARY OF THE INVENTION

Present invention relates to a novel non-digestible rare sugars-based oligosaccharide of formula **I** and the biosynthesis thereof through a transfructosylation reaction catalyzed by a levansucrase from *Bacillus subtilis* CECT 39, wherein the rare sugars of formula **I** have been demonstrated to be potential acceptors to produce novel carbohydrates, whilst molecular dynamics simulations further verified the feasibility of these substrates and enzyme binding. *In vitro* studies demonstrating the resistance to intestinal digestion and prebiotic properties of the rare sugar-based oligosaccharides of formula **I** provided insights about its structure-function relationship. The present invention consequently discloses novel biosynthesized carbohydrates of formula **I** with appealing functional and structural properties which make them useful as prebiotics and low caloric sweeteners.

Accordingly, a first aspect of the present invention relates to a compound of formula **I:**

[β-D-fructofuranoside(2→6)]ₙ-β-D-fructofuranoside-(2→X)-R Formula **I**

wherein:
R is D-tagatopyranosyl or L-sorbopyranosyl;
X is 1when R is α-D-tagatopyranosyl; or
X is 5 when R is L-sorbopyranosyl; and
n is an integer from 0 to 3.

In another embodiment the invention relates to the compound of formula **I** as defined above, selected from: 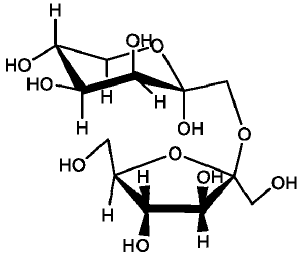 β -D-fructofuranosyl-(2→1)-α-D-tagatopyranose **(1α);** 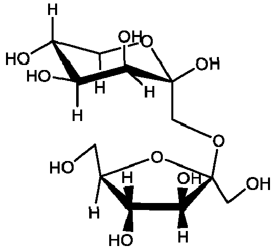 β -D-fructofuranosyl-(2→1)-β-D-tagatopyranose **(1β);** 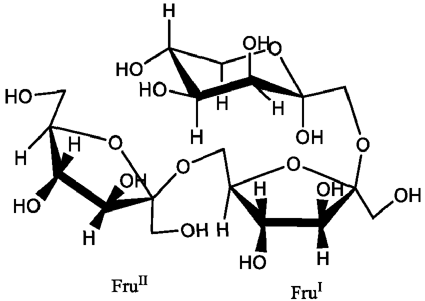 β -D-fructofuranosyl-(2→6)-β-D-fructofuranosyl-(2→1)-α-D-tagatopyranose **(2α);** 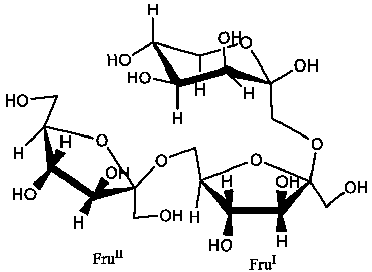 β -D-fructofuranosyl-(2→6)-β-D-fructofuranosyl-(2→1)-β-D-tagatopyranose **(2β);** 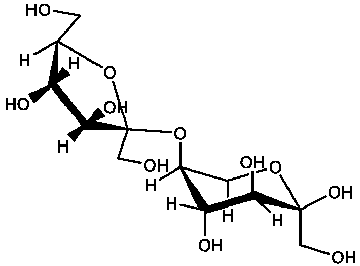 β -D-fructofuranosyl-(2→5)-α-L-sorbopyranose **(3);** 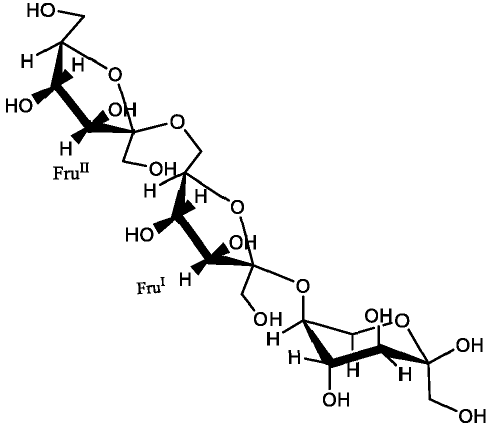 β -D-fructofuranosyl-(2→6)-β-D-fructofuranosyl-(2→5)-α-L-sorbopyranose **(4);** 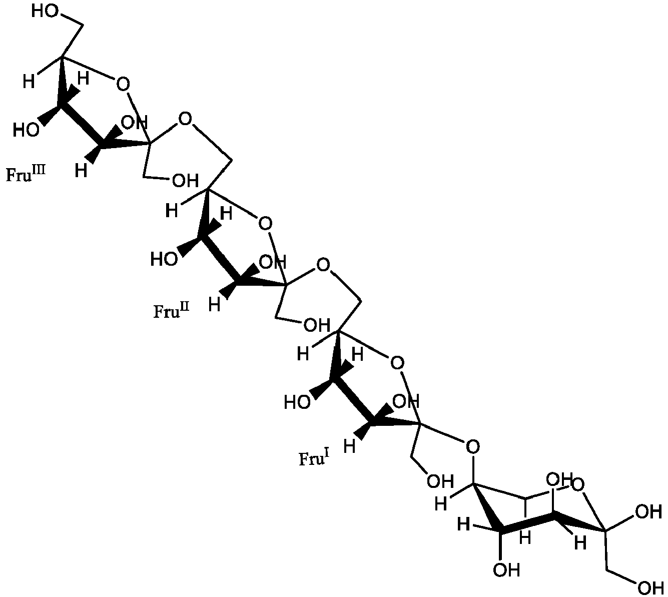 β -D-fructofuranosyl-(2→6)-β-D-fructofuranosyl-(2→6)-β-D-fructofuranosyl-(2→5)-α-L-sorbopyranose **(5);** and 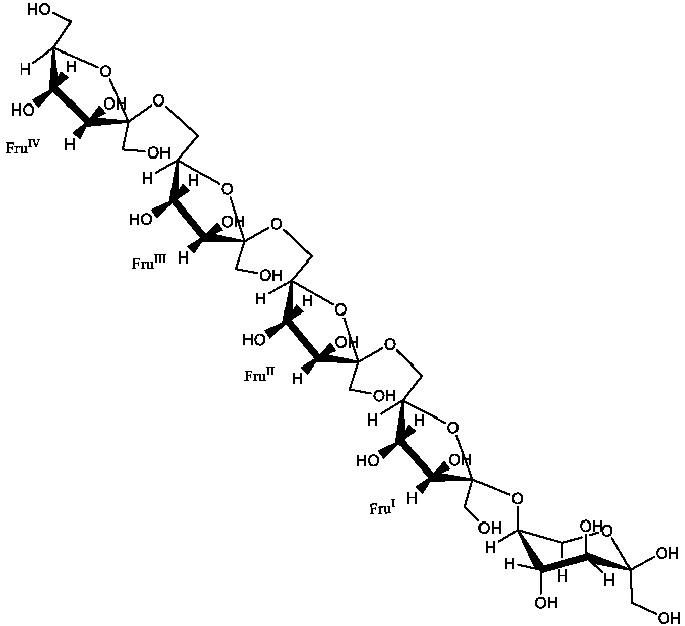 β -D-fructofuranosyl-(2→6)-β-D-fructofuranosyl-(2→6)-β-D-fructofuranosyl-(2→6)-β-D-fructofuranosyl-(2→5)-α-L-sorbopyranose **(6).**

Present invention demonstrates that the use of enzymes with transglycosidase activity aimed at tailoring rare sugars through its selective elongation could provide novel carbohydrates of formula **I** with a higher functionality than the tagatose itself.

Thus, another aspect of the invention relates to a process for obtaining a compound of formula I, which comprises:
i) Hydrolysis of sucrose (fructosyl donor) catalysed by the levansucrase from B. *subtilis* CECT 39 (SacB);
ii) transfructosylation of a saccharide of formula **II** and the hydrolysed sucrose obtained in step (i) to obtain a compound of formula **III** (a compound of formula **I** wherein n is 0):

   R-H (formula **II)**

   β -D-fructofuranoside-(2→X)-R (formula **III)**

   wherein:
   R is D-tagatopyranosyl or L-sorbopyranosyl; and
   X is 1when R is α-D-tagatopyranosyl; or
   X is 5 when R is L-sorbopyranosyl, and
iii) optionally, the polymerization between the compound of formula **III** obtained in step (ii) and from 1 to 3 hydrolysed sucrose as the one obtained in step (i) to obtain a compound of formula **I** wherein n is 1 to 3.

The compound of formula **III** defined above is a compound of formula **I** wherein n is 0.

Another aspect of the invention relates to the use of a compound of formula **I** as defined above, as a prebiotic or a low caloric sweetener.

Along the present description, the term "prebiotic" refers a substrate that is selectively utilized by host microorganisms conferring a health benefit.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and sequence listing are provided by way of illustration and are not intended to be limiting of the present invention.

### Examples

### Materials and methods

### Chemicals, reagents, and carbohydrates

All chemicals and reagents used were of analytical grade and purchased from Sigma-Aldrich (St Louis, MO, USA), VWR (Barcelona, Spain) and Merck (Darmstadt, Germany). D-Tagatose was purchased from Carbosynth (Compton, UK). Ultrapure water produced in-house with a laboratory water purification system (Milli-Q Synthesis A10, Millipore, Billerica, MA, U.S.A.) was used throughout.

*Production, Purification, and Activity Assay of Recombinant Levansucrase Enzyme.* Levansucrase (EC 2.4.1.10) from *Bacillus subtilis* CECT 39 (ATCC 6051) (SacB) was overproduced in *Escherichia coli and* purified. The total activity of levansucrase was expressed as the amount of free glucose, while the amount of formed fructose was measured for the determination of the hydrolytic (fructosidase) activity.

The transfructosylation activity (transferred fructose) was defined as the difference between the amount of released glucose and fructose. In consequence, the levansucrase expressed a total specific activity of 20.77 units per milligram (U mg⁻¹), where 1 unit is defined as the amount of enzyme releasing 1 µmol of glucose per minute at 37 °C and a sucrose concentration of 100 g L⁻¹ at pH 6.0 in 50 mM potassium phosphate buffer. The specific fructosidase activity was 10.79 U mg⁻¹, where 1 unit is defined as the amount of enzyme releasing 1 µmol of fructose per minute under the assayed conditions. Finally, the transfructosylation activity was 9.98 U mg⁻¹, where 1 unit is defined as the amount of enzyme required to transfer 1 µmol of fructose per minute at other molecules.

### Enzymatic synthesis of rare sugars-based oligosaccharides.

The production of rare sugars-based carbohydrates in the presence of sucrose (fructosyl donor) and D-tagatose or L-sorbose (acceptors) through a transfructosylation reaction catalyzed by the levansucrase from *B. subtilis* CECT 39 was carried out under the reaction conditions (i.e., pH 6.0 in 50 mM potassium phosphate buffer and 37 °C) and at concentration ratios of D-sucrose : D-tagatose or L-sorbose concentration ratios of 200 : 200, 200 : 300, 300 : 300, and 200 : 450g L⁻¹. The levansucrase (SacB) charge was also optimized by testing three different concentrations (that is, 0.6, 3.1 and 6.2 U mL⁻¹).

### Purification and isolation of D-tagatose- and L-sorbose-based oligosaccharides

Considering the lack of commercially available standard for rare sugars-based oligosaccharides, the main di- and trisaccharides synthesized after 24 h of transfructosylation reaction based on D-sucrose : D-tagatose mixture catalyzed by levansucrase under the optimized conditions described above were isolated and purified by preparative LC-RID on an Agilent Technologies 1260 Infinity LC System (Boeblingen, Germany) using a Zorbax NH₂ PrepHT preparative column (250 × 21.2 mm, 7 µm particle size) (Agilent Technologies, Madrid, Spain). Two mL of reaction mixtures (200 mg of total carbohydrates) were eluted with acetonitrile:water (65:35, v:v) as the mobile phase at a flow rate of 21.0 mL min⁻¹ for 30 min. The separated diand trisaccharides were collected using an Agilent Technologies 1260 Infinity preparative-scale fraction collector (Boeblingen, Germany), and the fractions were pooled, evaporated in a rotatory evaporator R-200 (Büchi Labortechnik AG, Flawil, Switzerland) below 25 °C and freeze-dried for subsequent NMR characterization and evaluation of their *in vitro* gastrointestinal digestibility and fermentation properties.

### Structural characterization of biosynthesized rare sugars-based oligosaccharides by Nuclear magnetic resonance (NMR)

Structure elucidation of the purified fractions was accomplished by Nuclear Magnetic Resonance spectroscopy (NMR). NMR spectra were recorded at 298 K, using D₂O as solvent, on an Agilent SYSTEM 500 NMR spectrometer (¹H 500 MHz, ¹³C 125 MHz) equipped with a 5-mm HCN cold probe. Chemical shifts of ¹H (δ_{H}) and ¹³C (δ_{C}) in parts per million were determined relative to internal standards of sodium [2, 2, 3, 3-²H₄]-3-(trimethylsilyl)-propanoate in D₂O (δ_{H} 0.00) and 1,4-dioxane (δ_{C} 67.40) in D₂O, respectively. One-dimensional (1D) NMR experiments (¹H and ¹³C{¹H}) were performed using standard pulse sequences.

### In vitro digestion of the main tagatose-based di- and trisaccharides using isolated pig small intestinal brush border membrane vesicles (BBMV).

Small intestinal digestion was carried out by dissolving the purified tagatose-based carbohydrates (4 mg) in 1.5 mL of small intestinal fluid (SIF). Briefly, SIF contained bile salts (10 mM), pancreatin (100 UmL⁻¹ trypsin activity), calcium chloride (0.6 mM), and other salts. In order to properly mimic the small intestinal digestive system, brushborder membranes from pig small intestine (BBMV) were isolated. Sixty milligrams of BBMV were added to the sample and incubated at 37 °C and 900 rpm in a Thermomixer^{®} (Eppendorf, Hamburg-Germany). Sample aliquots were taken after 1, 2 and 3h of hydrolysis and analyzed by GC-FID.

The main enzymatic activities of BBMV were estimated by individually dissolving, lactose, maltose, sucrose, isomaltose, isomaltulose and trehalose in PBS (0.2% w/v). One mililiter of each solution was transfer, in triplicate, to a microtube (2mL) containing 10 mg of BBMV. The mixture was incubated at 37 °C at 900 rpm (Thermomixer^{®}, Eppendorf, Hamburg-Germany). Fifty microliter aliquots were taken every 30 min, up to 3h of incubation. Each aliquot was then incubated, during 5 min, in a water bath at 95 °C to inactivate the enzymatic mixture and centrifuged at 8,000 xg (Mini Spin Eppendorf centrifuge, Hamburg, Germany). Then the supernatant was analysed by GC-FID as described above. The specific enzymatic activity (U/g) was expressed as µmol min-1 g-1 of total protein, where one unit is defined as the amount of total protein present in BBMV to hydrolyse 1 µmol of the corresponding disaccharide in one minute. Thus, the enzymatic activities were: β-galactosidase: 0.025 U mg-1; maltase: 0.448 U mg-1; sucrase: 0.035 U mg-1; trehalase: 0.033 U mg-1; isomaltase: 0.024 U mg-1; palatinase/isomaltulase: 0.024 U mg-1.

### Example 1: Synthesis and characterization of compounds 1 to 6

All reaction conditions disclosed in the Materials and Methods section showed the transfructosylation of the rare sugars catalyzed by SacB, although at different extent. The conditions which led to the highest yield of the novel oligosaccharides (compounds 1 to 6) contained in formula **I** were as follows: starting concentrations of sucrose and rare sugars (D-tagatose or L-sorbose) (300 g liter⁻¹ each) and levansucrase SacB charge (3.1 U ml⁻¹) in 50 mM potassium phosphate buffer (pH 6.0) at a temperature of 37 °C. The total yield (expressed as grams per 100 g of starting rare sugar added) of compounds 1 to 6 was optimized up to 25%.

All synthesized compounds were characterized by Nuclear Magnetic Resonance (NMR) by the combined use of 1D and 2D [¹H, ¹H] and [¹H, ¹³C] NMR experiments (gCOSY, TOCSY, multiplicity-edited gHSQC, gHMBC and hybrid experiment gHSQC-TOCSY). ¹H and ¹³C NMR chemical shifts observed are summarized below.

### β-D-fructofuranosyl-(2→1)-α-D-tagatopyranose (1α):

¹H-NMR (D₂O, 500 MHz) δ 3.60 (H6_{A}), 3.66 (H1_{A}, H1_{B}, H1'_{A}), 3.69 (H6'_{A}), 3.73 (H1'_{B}, H6_{B}), 3.79 (H6'_{B}), 3.83 (H4, H5), 3.87 (H3, H5'), 4.11 (H4'), 4.18 (H3').

¹³C-NMR (D₂O, 100 MHz) δ: 60.61 (C1'), 62.92 (C6, C6'), 64.63 (C1), 66.95 (C5), 70.83 (C3), 71.36 (C4), 74.96 (C4'), 77.61 (C3'), 81.82 (C5'), 98.53 (C2), 104.19 (C2').

### β-D-fructofuranosyl-(2→1)-β-D-tagatopyranose (1β):

¹H-NMR (D₂O, 500 MHz) δ 3.57 (H1_{A}), 3.65 (H1'_{A}, H6_{A}, H6_{B}), 3.67 (H6'_{A}), 3.73 (H1'_{B}), 3.80 (H6'_{B}), 3.86 (H5'), 3.89 (H5), 3.90 (H3), 3.99 (H4), 4.10 (H4'), 4.17 (H1_{B}), 4.18 (H3').

¹³C-NMR (D₂O, 100 MHz) δ: 60.78 (C1), 60.80 (C1'), 62.68 (C6'), 64.08 (C6), 64.63 (C5), 69.86 (C3), 71.45 (C4), 74.85 (C4'), 77.61 (C3'), 81.80 (C5'), 98.75 (C2), 104.25 (C2').

### β-D-fructofuranosyl-(2→6)-β-D-fructofuranosyl-(2→1)-α-D-tagatopyranose (2α):

¹H-NMR (D₂O, 500 MHz) δ 3.62 (H6_{A}), 3.63 (H6"_{A}, H6"_{B}), 3.66 (H1_{A}, H1_{B}, H1'_{A}, H1"_{A}), 3.69 (H6'_{A}), 3.75 (H1'_{B}), 3.77 (H6_{B}), 3.78 (H1"_{B}), 3.84 (H4), 3.85 (H5), 3.86 (H5"), 3.89 (H3), 3.92 (H6'_{B}), 3.94 (H5'), 4.10 (H4'), 4.11 (H4"), 4.17 (H3', H3").

¹³C-NMR (D₂O, 100 MHz) δ: 60.61 (C1'), 60.76 (C1"), 62.94 (C6), 63.06 (C6"), 63.58 (C6'), 64.71 (C1), 66.96 (C5), 70.73 (C3), 71.38 (C4), 75.21 (C4"), 75.76 (C4'), 77.26 (C3"), 77.43 (C3'), 80.79 (C5'), 81.77 (C5"), 98.59 (C2), 104.36 (C2"), 104.51 (C2').

### β-D-fructofuranosyl-(2→6)-β-D-fructofuranosyl-(2→1)-β-D-tagatopyranose (2β):

¹H-NMR (D₂O, 500 MHz) δ 3.59 (H1_{A}), 3.63 (H6"_{A}, H6"_{B}), 3.65 (H6_{A},H6_{B}), 3.66 (H1"_{A}), 3.69 (H6'_{A}), 3.78 (H1"_{B}), 3.86 (H5"), 3.92 (H3, H5, H6'_{B}), 3.94 (H5'), 4.01 (H4), 4.08 (H4'), 4.11 (H4"), 4.17 (H3"), 4.20 (H1_{B}).

¹³C-NMR (D₂O, 100 MHz) δ: 60.48 (C1'), 60.70 (C1), 60.76 (C1"), 63.06 (C6"), 63.53 (C6'), 63.98 (C6), 64.47 (C5), 69.92 (C3), 71.52 (C4), 75.21 (C4"), 75.64 (C4'), 77.26 (C3"), 77.32 (C3'), 80.86 (C5'), 81.77 (C5"), 98.82 (C2), 104.36 (C2"), 104.53 (C2').

### β-D-fructofuranosyl-(2→5)-α-L-sorbopyranose (3):

¹H-NMR (D₂O, 500 MHz) δ 3.51 (H1_{A}), 3.54 (H3), 3.67 (H6'A, H6'_{B}), 3.69 (H1_{B}), 3.73 (H4), 3.77 (H1'_{A}, H1'_{B}), 3.82 (H5, H5'), 3.92 (H6_{A}, H6_{B}), 4.00 (H4'), 4.17 (H3').

¹³C-NMR (D₂O, 100 MHz) δ: 61.48 (C1'), 62.18 (C6), 63.13 (C6'), 64.04 (C1), 70.77 (C3), 71.41 (C5), 72.95 (C4), 74.97 (C4'), 77.58 (C3'), 81.42 (C5'), 98.10 (C2), 104.15 (C2').

### β-D-fructofuranosyl-(2→6)-β-D-fructofuranosyl-(2→5)-α-L-sorbopyranose (4):

¹H-NMR (D₂O, 500 MHz) δ 3.51 (H1_{A}), 3.54 (H3), 3.61 (H6'_{A}), 3.66 (H6_{A}, H6"_{A}), 3.67 (H1"_{A}), 3.69 (H1_{B}), 3.74 (H4), 3.76 (H1'_{A}, H1'_{B}), 3.79 (H1"_{B}, H5), 3.82 (H6"_{B}), 3.86 (H5"), 3.88 (H6_{B}), 3.91 (H5'), 3.97 (H6'_{B}), 4.02 (H4'), 4.10 (H4"), 4.17 (H3', H3").

¹³C-NMR (D₂O, 100 MHz) δ: 60.79 (C1"), 61.22 (C1'), 62.20 (C6), 63.02 (C6"), 63.71 (C6'), 64.04 (C1), 70.73 (C3), 71.64 (C5), 72.89 (C4), 75.17 (C4"), 75.49 (C4'), 77.14 (C3"), 77.48 (C3'), 80.62 (C5'), 81.73 (C5"), 98.11 (C2), 104.47 (C2"), 104.53 (C2').

### β-D-fructofuranosyl-(2→6)-β-D-fructofuranosyl-(2→6)-β-D-fructofuranosyl-(2→5)-α-L-sorbopyranose (5):

¹H-NMR (D₂O, 500 MHz) δ 3.51 (H1_{A}), 3.54 (H3), 3.56 (H6"_{A}), 3.62 (H6'_{A}), 3.64 (H6‴_{A}), 3.65 (H1‴_{A}), 3.66 (H6_{A}), 3.69 (H1_{B}), 3.74 (H4), 3.77 (H1'_{A}, H1'_{B}, H1"_{A}, H1"_{B}), 3.79 (H5), 3.81 (H1‴_{B}, H6‴_{B}), 3.86 (H5‴, H6_{B}), 3.91 (H5', H6'_{B}), 3.93 (H6"_{B}), 3.94 (H5"), 4.02 (H4'), 4.10 (H4"), 4.11 (H4‴), 4.17 (H3‴), 4.18 (H3', H3").

¹³C-NMR (D₂O, 100 MHz) δ: 60.68 (C1‴), 61.23 (C1', C1"), 62.22 (C6), 63.10 (C6‴), 63.74 (C6'), 63.95 (C6"), 64.05 (C1), 70.75 (C3), 71.63 (C5), 72.89 (C4), 75.21 (C4‴), 75.44 (C4'), 75.95 (C4"), 77.07 (C3"), 77.08 (C3‴), 77.41 (C3'), 80.56 (C5'), 80.86 (C5"), 81.80 (C5‴), 98.10 (C2), 104.45 (C2‴), 104.53 (C2'), 104.84 (C2").

### β-D-fructofuranosyl-(2→6)-β-D-fructofuranosyl-(2→6)-β-D-fructofuranosyl-(2→6)-β-D-fructofuranosyl-(2→5)-α-L-sorbopyranose (6):

¹H-NMR (D₂O, 500 MHz) δ 3.51 (H1_{A}), 3.54 (H3), 3.56 (H6"_{A}, H6‴_{A}), 3.62 (H6'_{A}), 3.64 (H6ʺʺ_{A}), 3.66 (H1ʺʺ_{A}, H6_{A}), 3.69 (H1_{B}), 3.74 (H4), 3.77 (H1'_{A}, H1'_{B}, H1"_{A}, H1"_{B}, H1‴_{A}, H1‴_{B}, H1ʺʺ_{B}), 3.79 (H5), 3.81 (H6ʺʺ_{B}), 3.86 (H5ʺʺ, H6_{B}), 3.91 (H5', H6'_{B}), 3.93 (H6"_{B}, H6‴_{B}), 3.94 (H5", H5‴), 4.02 (H4'), 4.10 (H4", H4‴), 4.11 (H4ʺʺ), 4.18 (H3', H3", H3‴, H3ʺʺ).

¹³C-NMR (D₂O, 100 MHz) δ: 60.61 (C1""), 61.23 (C1', C1", C1‴), 62.15 (C6), 63.09 (C6ʺʺ), 63.99 (C1, C6', C6", C6‴), 70.74 (C3), 71.62 (C5), 72.89 (C4), 75.22 (C4ʺʺ), 75.39 (C4'), 75.99 (C4", C4‴), 76.96 (C3ʺʺ), 77.09 (C3", C3‴), 77.42 (C3'), 80.56 (C5'), 80.83 (C5"), 80.94 (C5‴), 81.76 (C5ʺʺ), 98.10 (C2), 104.45 (C2""), 104.54 (C2'), 104.84 (C2", C2‴).

### Example 2: In vitro digestibility by pig BBMV disaccharidases

Sucrose was used as a positive control of the *in vitro* digestion system using pig BBMV and, as expected, was readily digested. Thus, sucrose was fully hydrolyzed after 1 hour of digestion (Table 1).

**Table 1. Determination of sucrose, compounds 1 and 2 during a simulated small intestinal digestion. Data are expressed as the mean with the SD in brackets (n = 3) and represent the percentage of carbohydrate determined against its initial concentration.**

| **Digestion time (h)** | **Sucrose** | **1** | **2** |
|---|---|---|---|
| 0 | 100.0 (3.5) | 100.0 (17.5) | 100.0 (0.5) |
| 1 | Not detected | 110.6 (17.3) | 88.3 (5.6) |
| 2 | Not detected | 104.8 (11.8) | 86.0 (4.6) |
| 3 | Not detected | 99.8 (17.7) | 78.0 (13.3) |

The hydrolysis/resistance degrees of compounds 1 and 2 were determined following their *in vitro* digestion of their purified forms isolated from the enzymatic reaction mixture. The obtained data indicated that the compound 1 was fully resistant and the compound 2 highly resistant to the hydrolytic action of the mucosal disaccharidases embedded in the pig small intestinal BBMV throughout the whole digestion time (3 hours), thus supporting their potential role as low calorie ingredients (Table 1). Moreover, the low or lack of digestibility of the biosynthesized tagatose-based carbohydrates warrants further studies dealing with their fermentable properties and potential ability to selectively modulate the gut microbiota.

## Claims

1. A compound of formula I:
[β-D-fructofuranoside(2→6)]ₙ-β-D-fructofuranoside-(2→X)-R Formula I
wherein:
R is D-tagatopyranosyl or L-sorbopyranosyl;
X is 1when R is α-D-tagatopyranosyl; or
X is 5 when R is L-sorbopyranosyl; and
n is an integer from 0 to 3.

2. The compound of formula **I** according to claim 1, selected from:
D-fructofuranosyl-(2→1)-D-tagatopyranose **(1)**
D-fructofuranosyl-(2→6)-D-fructofuranosyl-(2→1)-D-tagatopyranose **(2);**
D-fructofuranosyl-(2→5)-L-sorbopyranose **(3);**
D-fructofuranosyl-(2→6)-D-fructofuranosyl-(2→5)-L-sorbopyranose **(4);**
D-fructofuranosyl-(2→6)-D-fructofuranosyl-(2→6)-D-fructofuranosyl-(2→5)-L-sorbopyranose **(5);** and
D-fructofuranosyl-(2→6)-D-fructofuranosyl-(2→6)-D-fructofuranosyl-(2→6)-D-fructofuranosyl-(2→5)-α-L-sorbopyranose **(6).**

3. A process for obtaining a compound of formula I according to any of claims 1 or 2, which comprises:
i) Hydrolysis of sucrose (fructosyl donor) catalysed by the levansucrase from B. *subtilis* CECT 39 (SacB);
ii) transfructosylation of a saccharide of formula **II** and the hydrolysed sucrose obtained in step (i) to obtain a compound of formula **III** (a compound of formula I wherein n is 0):
R-H (formula **II)**
β -D-fructofuranoside-(2→X)-R (formula **III)**
wherein:
R is D-tagatopyranosyl or L-sorbopyranosyl; and
X is 1when R is α-D-tagatopyranosyl; or
X is 5 when R is L-sorbopyranosyl, and
iii) optionally, the polymerization between the compound of formula III obtained in step (ii) and from 1 to 3 hydrolysed sucrose as the one obtained in step (i) to obtain a compound of formula I wherein n is 1 to 3.

4. Use of a compound of formula **I** according to any of claims 1 or 2, as a prebiotic or a low caloric sweetener.
